# EUROPEAN PATENT APPLICATION

(11) **EP 2 135 612 A1**
(43) Date of publication of application: **23.12.2009**
(21) Application number: 08011244.4
(22) Date of filing: 20.06.2008
(51) Int. Cl.: A61K 31/708, A61K 9/00

(54) **Use of ATP in the preparation of a medicament for treating, alleviating and/or enhancing survival of pre-terminal cancer patients**

(71) Applicant: Universiteit van Maastricht, 6200 MD Maastricht (NL)
(72) Inventor: The designation of the inventor has not yet been filed
(74) Representative: Huygens, Arthur Victor

(57) **Abstract**

The present invention relates to the use of ATP for the preparation of a medicament, in particular in the form of intravenous infusions, for treating, alleviating and/or enhancing survival of pre-terminal cancer patients. In the investigated population of pre-terminal cancer patients, ATP infusions, at the dose and schedule studied, had a favorable effect on triceps skin fold thickness and survival, especially in weight-stable patients and patients with lung cancer.

## Description

### Field of the invention

The present invention relates to the use of adenosine 5'-triphosphate (ATP) in the preparation of a medicament, in particular in the form of infusions for treating, alleviating and/or enhancing survival of pre-terminal cancer patients. The inventors investigated the effect of intravenous infusions of adenosine 5'-triphosphate (ATP) on nutritional status and survival in pre-terminal cancer patients and achieved surprising results which are herein described and embodied in the present invention.

### Introduction

Cachexia is one of the most common and devastating symptoms in patients with advanced cancer (1, 2) ranging from 24% early at the diagnosis of advanced cancer to more than 80% at the terminal stages (1). The presence of weight loss is associated with decreased survival (3-7), and in some cases, excessive weight loss may itself be the cause of death (8). Cytokines are thought to play a pivotal role in the development and progression of cancer cachexia (9-12). So far, neither nutritional supplementation (1, 9, 13) nor pharmacological interventions (9, 11, 12, 14, 15) have been able to reverse cachexia. Research on novel approaches to counteract cancer cachexia therefore remains highly relevant.

A potential agent in the treatment of cancer-cachexia is adenosine 5'-triphosphate (ATP). A randomized clinical trial (RCT) in patients with non-small-cell lung cancer (NSCLC) (16, 17) showed that ATP infusions, given over 30 h at 2- to 4-week intervals (infusion rate max. 75 µg/kg.min), had marked beneficial effects on nutritional intake, body weight, fat and muscle mass. ATP treatment was also associated with a highly significant survival benefit in weight-losing patients with stage IIIB NSCLC (18). Moreover, ATP prevented the progressive increase in plasma concentrations of C-reactive protein (CRP) and the decrease in albumin which were seen in the control group (16), providing a possible mechanism underlying the favorable effects of ATP on the nutritional status in these patients. The anti-inflammatory properties of ATP were recently confirmed by Swennen et al. (19, 20) who, using an ex vivo model of stimulated whole blood, demonstrated that ATP inhibited TNF-α and enhanced IL-10 release (19) via stimulation of the P2Y₁₁ and P2Y₁₂ receptor, respectively (20).

Based on these promising results, we initiated a randomized clinical trial (RCT) to investigate the effects of ATP (8-hr infusions once per week for 8 weeks) on the nutritional status in patients with different types of cancer in the pre-terminal stage, defined as a life expectancy <6 months. Based on the earlier observed survival benefit of ATP, we also investigated the effect of ATP on short term survival (i.e. during the 8-week ATP intervention) as well as long term survival (0-6 months, based on the maximum life expectancy of 6 months in the study population).

### Summary of the invention

The present invention is based on the following findings.

Ninety-nine pre-terminal cancer patients (estimated life expectancy 1-6 mo) with mixed tumor types were randomly allocated to receive either ATP i.v. weekly (8 h/wk, max. 50 µg/kg.min) for 8 weeks, or no ATP (control group). Nutritional status parameters were assessed until 8 weeks, and analyzed by repeated-measures analysis of covariance. Cox proportional hazards models were fitted to assess the effect of ATP on short term (0-8 weeks) and long term (0-6 months) survival.

Fifty-one patients were randomized to ATP and 48 to the control group. Results showed a significant favorable effect of ATP on triceps skin fold thickness (between-group difference per 8 weeks: 1.76 mm, 95% Confidence interval (CI): 0.48 to 3.12 mm; p=0.009), and a significant difference in short-term survival (0-8 weeks) in favor of the ATP group (HR: 0.40, 95% CI: 0.17- 0.95; p=0.037), and in weight-stable as well as in lung cancer patients, long term survival (0-6 months) was also significantly better in ATP-treated patients (weight stable patients: HR: 0.40, 95% Cl: 0.19 - 0.83; p=0.014; patients with lung cancer: HR: 0.35, 95% Cl: 0.14 - 0.88; p=0.025).

In this population of pre-terminal cancer patients, ATP infusions, at the dose and schedule studied, had a favorable effect on triceps skin fold thickness and survival, especially in weight-stable patients and patients with lung cancer. Studies are urgently needed to define the effect of ATP on survival and tumor growth in specific types of cancer, including combination of ATP with curative treatment.

In one aspect of the invention, ATP infusions are prepared in a manner known in the art for administration, in particular intravenous infusion, to a subject suffering from cancer at a pre-terminal stage, who is in need of such therapy. The dose and schedule of administration will depend on usual factors such as nature and severity of the disease, weight, age and sex of the subject and may be determined by a skilled practitioner based on his/her experience and on the present disclosure without exercising undue experimentation. The present invention further relates to pharmaceutical compositions, in particular infusions, comprising ATP in an effective amount for treating and enhancing survival of pre-terminal cancer patients.

### Brief description of the drawings

**Figure 1****.** Flow diagram of randomized patients.
**Figure 2****.** Kaplan-Meier plots of survival in patients treated with ATP versus no ATP (control): **(A)** total group (ATP: n=51; control: n=48), **(B)** patients with <5% weight loss over the previous 6 months (ATP: n=33; control: n=31), **(C)** patients with lung cancer (ATP: n=21; control: n=23).

### Patients, materials and methods

### Patients

From March 2002 through October 2006, 100 patients were included in the study. The Departments of Medical Oncology and Pulmonology in five centra in the southern half of the Netherlands and 50 general practitioners participated in patient recruitment.

Eligible were patients with histologically or cytologically proven cancer, without curative treatment options, with an estimated life expectancy of one to six months, a World Health Organization performance status 1 or 2, suffering from at least one of the following complaints: fatigue, weight loss >5% over the previous six months, or anorexia. Patients with symptomatic angina pectoris, symptomatic heart failure or any form of AV-block (assessed by ECG), as well as patients with cognitive dysfunction, or concurrent palliative chemotherapy were excluded. The study was approved by the Ethical Committees of all hospitals involved in the study, and all patients signed written informed consent prior to the study.

### Study design

After baseline measurements, patients were stratified for center, tumor type, and weight loss (≤5% vs. >5% over past 6 months), and then randomly assigned to receive either usual care, standard nutritional advice and ATP (ATP group), or usual care and standard nutritional advice alone (control) group), using computer-generated random numbers with permutation blocks of four. In both groups, nutritional status parameters and co-interventions were assessed until 8 weeks.

### ATP intervention

Patients allocated to the ATP group received weekly ATP infusions during 8-10 hours over a period of 8 weeks (21). The first ATP infusion was offered under medical supervision at the day care center of the participating hospitals. ATP infusions were started at a dose rate of 20 µg/kg·min and increased in steps of 10 µg/kg·min per 10 minutes, until reaching a maximum dose rate of 50 µg/kg·min, or the maximally tolerated dose (MTD) if this was lower, in case of side effects. Thereafter, ATP was Infused at a continuous rate. Subsequent infusions were given at MTD in the patient's home.

### Outcome assessment

Body height was determined using a flexible measuring tape. Body weight was measured without shoes, using an electronic scale (Soehnle 7407 Translucia, Germany). Triceps skin fold thickness was measured in triplicate at the dominant arm, with a Holtain® skin fold caliper (CMS weighing equipment LTD, London UK), and the median used for further calculations. Mid-upper arm circumference was measured in duplicate at the dominant side with a flexible measuring tape, and the mean value was used for further calculations.

In order to exclude inter-observer variability, longitudinal measurements in one patient were performed by one observer. Nutritional intake was assessed by a 3-day food diary, including meal preparation details, and checked by an experienced dietician. Intake of energy and protein was calculated using the Dutch Food Composition Table 2001 (NEVO, The Hague, The Netherlands), by the Software Program Komeet (version 4.0.58; BaS Nutrition Software, Arnhem, The Netherlands).

### Statistical analysis

Data were doubly entered and analyzed according to the intention-to-treat principle. Curves were fitted to describe the time course of nutritional status parameters, and differences over time between the two groups appraised by repeated measurement analysis of covariance, using SAS Proc Mixed version 9.1 (SAS Institute Inc., Cary, NC, USA). Independent variables were the treatment indicator variable, the baseline measurement, measurement time, and the interaction between time and treatment. Statistical significance of treatment effects was assessed by testing the interaction between time and treatment. For weight analyses, patients with oedema and/or ascites were excluded. Because of the favorable previous results in patients with lung cancer (17), all analyses were repeated for this subgroup separately.

Cox regression models were fitted including interaction terms between treatment and dummy variables for short term (i.e. 0-8 wks) and long term survival (i.e. 0-6 mo; dummy variables for 0-8 wks and 8 wks-6 mo were combined into a single dummy variable (0-6 mo) since Cox proportional hazards assumption was tested and not rejected). Median survival time was determined by extrapolation from confounder-adjusted Kaplan Meier plots. Because of previously shown survival benefit of ATP treatment in weight-losing stage IIIB NSCLC patients (18), we also performed stratified analyses in weight-stable and weight-losing patients separately defined as ≤5% or >5% weight loss over the past 6 months (22).

All models were appropriately adjusted for confounders. Two-tailed p-values <0.05 were considered statistically significant.

### Results

### Study population

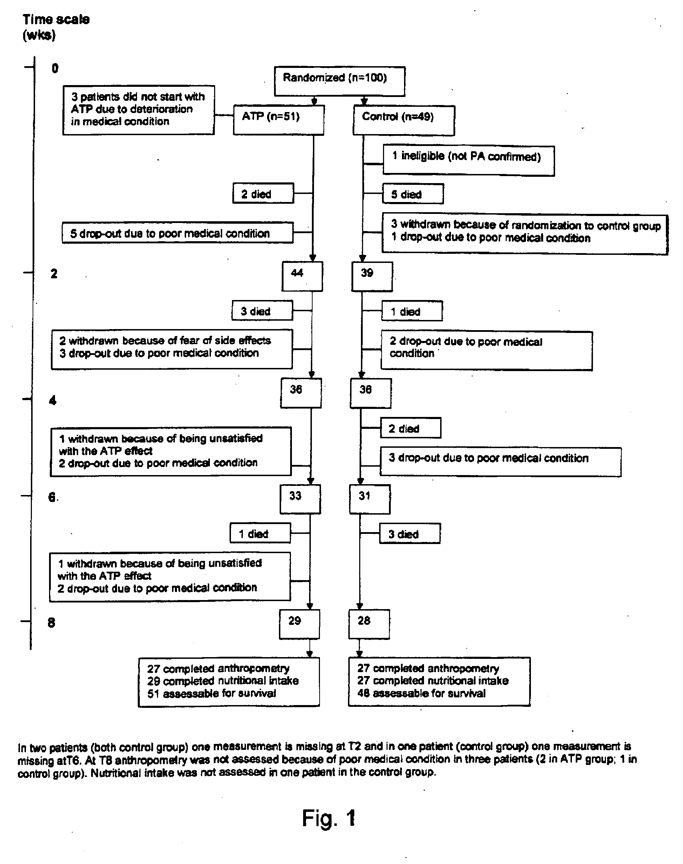

The flow diagram of the study is shown in Fig. 1. One hundred patients were randomly assigned to the ATP (n=51) or control (n=49) group. One patient in the control group was excluded from the analysis because cancer was not confirmed histologically or cytologically. Sixteen patients (7 ATP, 9 controls) died or dropped out before the first follow-up measurement, leaving 83 assessable patients (44 ATP, 39 controls). Fifty-seven patients (29 ATP, 28 control) completed the full 8-week study period. Total drop-out rates were equally distributed over ATP (n=22) and control patients (n=20). Reasons for drop-out were death in six ATP and 11 control patients, deterioration in medical condition in 12 ATP and six control patients, and withdrawal in four ATP and three control patients.

### Baseline characteristics

Mean age of the total study population was 66.4 years, and 66% of the patients were male. Lung cancer was the most frequent tumor type (45%), followed by gastro-intestinal cancers (25%), especially colon cancer (13%). Seventy percent of the patients had WHO performance index 1, 30% WHO 2.

As shown in Table 1 below, there was some unbalance between the randomization groups in favor of the control group: randomized and assessable patients in the ATP group had a worse performance status, a lower energy intake, a higher frequency of oedema and ascites. Also, ATP patients had lost more body weight on average and had a higher proportion of patients with ≥10% weight loss.

**Table 1. Baseline patient characteristics of randomly assigned and assessable patients**

| | | **ATP** | | **Control** | |
|---|---|---|---|---|---|
| | | Randomly assigned [1] | Assessableⁿ [2] | Randomly assigned [1] | Assessableⁿ [2] |
| | | (N=51) | (N=44) | (N=49) | (N=39) |
| | | N (%) or | N (%) or | N (%) or | N (%) or |
| | | Mean ± SD | Mean ± SD | Mean ± SD | Mean ± SD |
| Sex | Male | 35 (69%) | 34 (77%) | 31 (63%) | 26 (67%) |
| | Female | 16 (31%) | 10 (23%) | 18 (37%) | 13 (33%) |
| Age (y) | | 68 ±9.8 | 68 ±9.6 | 65 ±10.5 | 65 ±11.0 |
| Years of cancer at inclusion | | 2.3 ± 2.7 | 2.5 ± 2.9 | 2.7 ± 3.3 | 2.7 ± 3.3 |
| Tumor type | Lung | 21 (41%) | 19 (43%) | 24 (49%) | 21 (54%) |
| | Colon | 8 (16%) | 8 (18%) | 5 (10%) | 3 (8%) |
| | Gastro-intestinal other | 6 (12%) | 3 (7%) | 6 (12%) | 4 (10%) |
| | Prostate | 5 (10%) | 5 (11%) | 4 (8%) | 3 (8%) |
| | Other | 11 (21%) | 9 (21%) | 10 (21%) | 8 (20%) |
| Smoking | Yes | 23 (45%) | 20 (455%) | 15 (31%) | 13 (33%) |
| | No | 28 (55%) | 24 (55%) | 33 (67%) | 25 (64%) |
| | Missing | - | - | 1 (2%) | 1 (3%) |
| WHO-performance index* | | | | | |
| | 1 | 33 (65%) | 30 (68%) | 37 (76%) | 33 (85%) |
| | 2 | 18 (35%) | 14 (32%) | 12 (24%) | 6 (15%) |
| Fatigue | | | | | |
| | Yes | 48 (94%) | 41 (93%) | 48 (98%) | 39 (100%) |
| | No | 3 (6%) | 3 (7%) | 1 (2%) | 0 (0%) |
| Loss of appetite | | | | | |
| | Yes | 36 (71%) | 31 (70%) | 31 (63%) | 23 (59%) |
| | No | 15 (29%) | 13 (30%) | 18 (37%) | 16 (41%) |
| EORTC QLQ-C30: | | | | | |
| Physical functioning | | 44.0 ±25.9 | 49.1 ± 23.9 | 45.3 ± 26.2 | 50.1 ± 24.7 |
| Fatigue | | 65.6 ± 27.0 | 62.9 ± 27.2 | 58.3 ±26.6 | 54.1 ± 24.3 |
| Appetite loss | | 49.7 ± 37.9 | 48.5 ± 37.7 | 43.1 ± 34.4 | 41.0 ± 34.6 |
| Energy intake (Mega-Joulelday) | | 6.6 ±2.1 | 6.8 ±2.1 | 7.4 ±2.3 | 7.7 ± 2.1 |
| Weight [3] | | 69.9 ±15.2 | 71.4 ±15.3 | 68.4 ±17.2 | 72.0 *16.9 |
| Body mass index (BMI) (kg/m²) [3] | | 23.3 ±4.5 | 23.7 ±4.8 | 23.1 ±4.6 | 24.1 ±4.4 |
| Weight change In previous 6 mo (%) | | -3.5 ± 7,9 | -2.8 ± 7.6 | -2.6 ± 9.0 | -1.0 ± 9.0 |
| | ≥5% weight loss in previous 6 mo | 18 (35%) | 13 (30%) | 17 (35%) | 10 (26%) |
| Overall weight change (%) [4] | | -10.9 ± 10.0 | -9.9 ± 10.0 | -9.1 ± 13.5 | -6.1 ±12.4 |
| | ≥ 10% overall weight loss | 27 (53%) | 22 (50%) | 20 (41%) | 12 (31%) |
| Oedema and/or ascites | | 14 (28%) | 12 (27%) | 8 (17%) | 6 (15%) |
| Oedema | | 12 (24%) | 11 (25%) | 8(17%) | 6 (15%) |
| Ascites | | 6 (12%) | 4 (9%) | 2 (4%) | 0 (0%) |
| Upper arm circumference (cm) | | 29.4 ±4.4 | 29.5 ±4.4 | 28.6 ±4.5 | 29.6 ±4.0 |
| Triceps skin fold thickness mm | | 15.3 ±6.9 | 15.1 ±6.8 | 16.4 ±8.2 | 17.6 ±7.9 |

| | | | | | |
|---|---|---|---|---|---|
| ⁿ At least one follow-up analysis was performed * WHO = World Health Organization [1] Data available for energy intake, n=49 (ATP) and n=48 (control), for upper arm circumference, n=51 (ATP) and n=47 (control), for triceps skin fold, n=50 (ATP) and n=46 (control) [2] Data available for energy intake, n=42 (ATP) and n=39 (control) [3] Patients with ascites or oedema excluded from analysis. Randomly assigned: for weight, n=27 (ATP) and n=31 (control), for BMI, n=25 (ATP) and n=31 (control) Assessable: for weight, n=22 (ATP) and n=24 (control), for BMI, n=21 (ATP) and n=24 (control) [4] Overall weight change = current weight minus stable pre-illness weight | | | | | |

### Intervention

Of the 51 patients assigned to the ATP-group, three patients (6%) did not start the infusion because of rapid deterioration of their condition. The remaining 48 patients received a mean of 5.5 ATP infusions per patient over the 8-week period. Twenty-four patients (47%) completed all eight ATP infusions. Of these 24 patients, 12 patients pledged to continue ATP administration after completion of the regular 8-week study period. Over the subsequent four months, these patients received a mean of 7 ATP infusions per patient (21).

### Co-interventlons

At baseline, 10 patients (5 ATP, 5 control group) were treated with hormones and 4 patients with gefitinib (2 ATP, 2 control); no patients were treated with palliative chemotherapy or radiotherapy at baseline. During the study, one patient (control) received palliative radiotherapy for skeletal metastases, and two patients (control) started with chemotherapy. Hormone therapy was continued in nine patients (5 ATP, 4 control) and reduced in one patient (control). Gefitinib was started in one patient (control), raised in one patient (ATP), and continued In three patients (1 ATP, 2 controls). Use of non-opioid or opioid analgesics, corticosteroids, antihypertensives, antiemetics, antidiarrhoics, laxatives, diuretics, antibiotics, antidiabetics or COPD drugs was similar in the ATP and control group (data not shown).

### Effect of ATP on nutritional status

Changes per week in anthropometric measurements during the 8-week intervention period in the ATP and control group are shown in Table 2 below. Both in ATP-treated and control patients, weight, mid-upper arm circumference and triceps skin fold thickness decreased. However, triceps skin fold thickness decreased significantly less in the ATP group (change per 8 weeks: -1.84 mm, 95% Cl: -3.92 to 0.24 mm) than in the control group (-3.60, 95% Cl: -5.84 to -1.36 mm; between-group difference: 1.76, 95% Cl: 0.48 to 3.12 mm, p=0.009). No statistically significant difference between the two groups was observed for weight, mid-upper arm circumference, or intake of energy and protein. In the subgroup of patients with lung cancer, results were similar (data not shown).

**Table 2. Changes per 8 weeks in anthropometric measurements in patients treated with ATP and control patients, and between-group difference. 95% Cl, 95% confidence interval.**

| | **ATP group** | | **Control group** | | **Between group difference** | |
|---|---|---|---|---|---|---|
| | **Estimate** | **95% Cl** | **Estimate** | **95% Cl** | **Estimate** | **95% Cl** |
| **Weight (kg)** | -2.62 | -6.16 to 0.96 | -2.20 | -6.24 to 1.92 | -0.42 | -2.24 to 1.40 |
| **Triceps skin fold thickness (mm)** | -1.84 | -3.92 to 0.24 | -3.60 | -5.84 to -1.36 | 1.76 | 0.48 to 3.12 |
| **Mid-upper arm circumference (cm)** | -2.24 | -3.36 to -1.20 | -1.52 | -2.80 to -0.24 | -0.72 | -1.52 to 0.08 |

| | | | | | | |
|---|---|---|---|---|---|---|
| Number of patients: weight: ATP, n=26; control, n=27; Triceps skin fold thickness ATP, n=48, control, n=45; mid-upper arm circumference: ATP, n=49: control, n=46. | | | | | | |

### Effect of ATP on survival

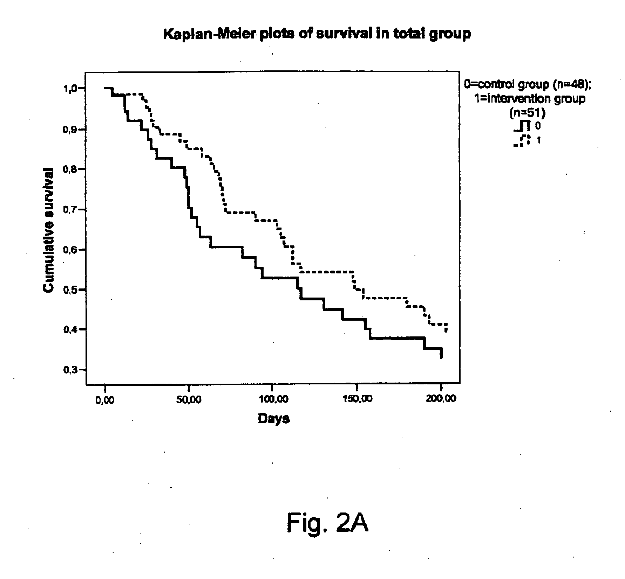

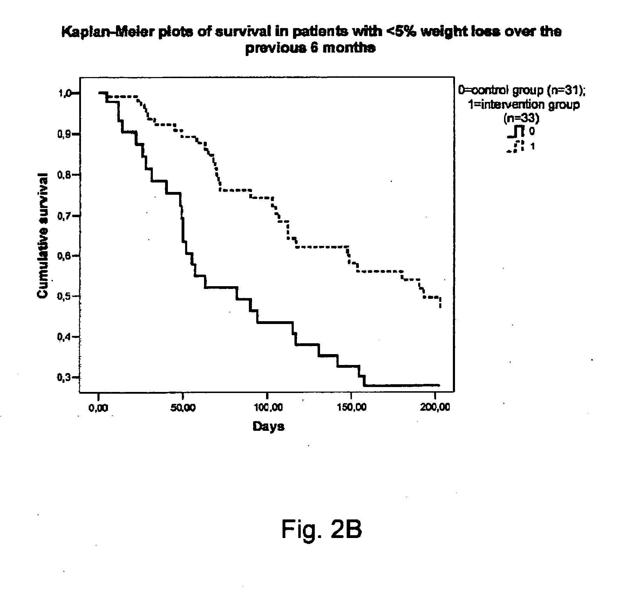

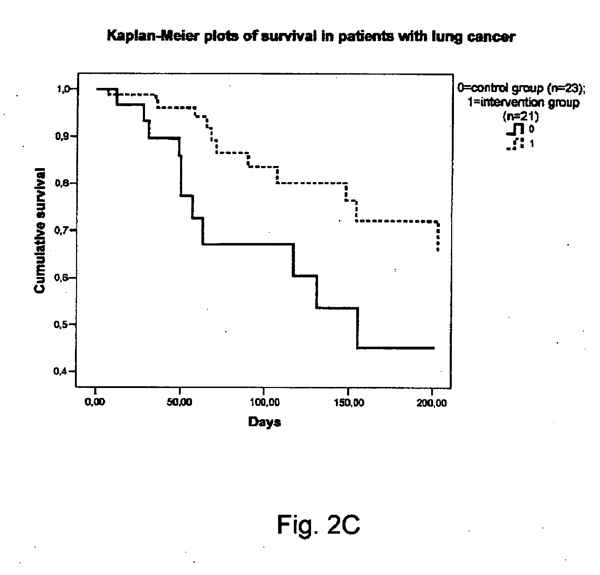

Kaplan Meier plots of survival in ATP-treated and control patients are given in Fig. 2. As shown in Fig. 2A, Cox regression revealed a statistically significant difference in short-term (0-8 weeks) survival in favor of ATP-treated patients (HR of ATP vs. control: 0.40, 95%Cl: 0.17-0.95; p=0.037). Long term survival (0-6 months) was not significantly different between the ATP and control group (HR: 0.71, 95%Cl: 0.40-1.28; p=0.26). Median survival was 150 days in the ATP group and 116 days in the control group. On the census date of 10 September 2007, nine patients (6 ATP, 3 control) were still alive.

Stratified analysis in weight-stable and weight-losing patients showed a significant survival benefit of ATP treatment in weight-stable patients (Fig. 2B; ATP: n=33; control: n=31), both during the 8-week intervention (HR: 0.20, 95%Cl: 0.08-0.54; p=0.001) and for long term survival (0-6 months; HR: 0.40, 95%Cl: 0.19-0.83; p=0.014). No significant effect of ATP on survival was found in weight-losing patients (data not shown).

In patients with lung cancer (n=44) (Fig. 2C), results also indicated a significant short and long term survival benefit of ATP treatment (0-8 weeks: HR: 0.17, 95%Cl: 0.04-0.78; p=0.23; 0-6 months: HR: 0.35, 95%Cl 0.14-0.88; p=0.025).

### Discussion

In the present RCT we investigated the effects of ATP infusions on the nutritional status and survival in patients with pre-terminal cancer of different tumor types. In view of the short life expectancy of this patient population (<6 months), we chose to apply a frequent ATP treatment schedule over a short period of time (i.e. weekly 8-hour infusions of ATP over 8 weeks). All analyses were adjusted for confounders.

Results showed a significant advantage in short term survival (0-8 weeks) for ATP-treated patients relative to control patients (HR 0.40), which was more pronounced in weight-stable patients (HR 0.20) and patients with lung cancer (HR 0.17). Moreover, ATP treatment even showed significant benefit for long term survival (0-6 months) in both weight-stable patients (HR 0.40) and patients with lung cancer (HR 0.35). This effect of ATP on survival is remarkable since our study population was composed of refractory cancer patients with pre-terminal illness with an estimated life expectancy <6 months.

The question could be raised why the observed survival benefit of ATP in our study population was especially present and lasted longer in weight-stable patients, as opposed to patients with recent weight loss. Recent weight loss is well-known to be a predictor of survival (3-7), presumably due to a higher tumor stage (23-25). Many malignant tumors produce humoral factors, such as cytokines or specific cachexia-inducing factors, thereby inducing weight loss (10-12, 26). Thus, the notion of better effects of ATP in weight-stable patients could indicate that ATP may be more effective in patients with less advanced disease. This hypothesis is supported by Agteresch et al. (18), who reported favorable effects of ATP on survival only in NSCLC patients with locally extended disease (stage IIIB), but not In patients with metastatic disease (stage IV).

However, of note, Agteresch et al. (18) only observed a significant favorable effect of ATP on survival in stage IIIB NSCLC patients with weight loss, but not in weight-stable stage IIIB NSCLC patients. Although this finding at first sight appears to contrast with our finding (i.e. we observed a stronger and longer lasting effect of ATP on survival in weight-stable patients), these paradoxal results can be explained by different definitions of weight loss applied in the two studies. Agteresch et al. (18) stratified their patients by the presence of overall loss >5% of body weight, i.e. long-terrn weight loss since the onset of the disease. Instead, in view of the relation between recent weight loss and sunrival (3), we stratified our subjects by recent weight loss, i.e. weight loss over the last 6 months (22). To check for a potential effect of the different definitions of weight loss in the two studies, we repeated our analyses in lung cancer patients using the same definition of weight loss as Agteresch et al. (18) (i.e. ≤5% vs. >5% overall weight loss)., Results showed an almost statistically significant survival benefit of ATP in lung cancer patients with overall weight loss (HR: 0.21, 95%Cl: 0.04-1.01; p=0.052), corroborating the results of Agteresch et al.(18).

The mechanism underlying the effect of ATP remains to be elucidated. Agteresch et al. (18) suggested that the effect of ATP on survival was mediated by the anticachectic effect of ATP in their study population. However, based on our finding of stronger and longer lasting effects of ATP on survival in weight-stable patients, it would appear more likely that other mechanisms underlie the favorable effect of ATP on survival, such as a direct effect of ATP on malignant cell growth (18, 27-31). Although to our knowledge no RCTs on the effects of ATP on tumor growth have been reported, a phase II study in 15 chemotherapy-naïve patients with stage IIIB/IV NSCLC patients performed by Mendoza and coworkers (32) showed stable disease in 8 patients after one to four 96-hour cycles of intravenous ATP (50-65 µg/kg.min), given at 4-week intervals, which would support a potential effect of ATP on tumor growth in NSCLC patients.

Our findings with regard to the effects of ATP on the nutritional status partly confirm results of the earlier RCT in patients with advanced NSCLC (16, 17). In this earlier trial, the favorable effects of ATP on triceps skin fold thickness came into effect within 8 weeks, whereas ATP effects on body weight arid mid-upper arm circumference only emerged after a delay of 8-16 weeks. The absence of significant effects of ATP on body weight and mid-upper arm circumference in the present study would suggest that our 8-week intervention in pre-terminal patients may have been too short to detect significant effects of ATP on the nutritional status. In combination with the earlier RCT, our data would suggest that the effects of ATP on fat mass may be substantially more rapid than the effects on body weight.

Some methodological issues need to be considered. First, due to the pre-terminal illness of our patient population, baseline data and time changes of outcome parameters showed high between-subject variability (SD), limiting the power of the study to detect effects of ATP on nutritional status. The high drop-out rate, which was unavoidable in this pre-terminal study population, further reduced the power of the study.

Second, co-interventions (chemotherapy, antibiotics, corticosteroids) could also have influenced patients' nutritional status or survival. However, the only difference between the control and·ATP group was the start of palliative radiotherapy in one control patient, palliative chemotherapy in two control patients and gefitinib in one control patient, all of which would therefore induce bias towards longer survival in the control group, and thus cannot explain our finding of a longer survival in ATP-treated patients.

Finally, for ethical reasons, our trial was not placebo-controlled. Since the belief that emotional state could affect survival exists among patients and professionals (33), the question might be raised whether the observed effect of ATP on survival in our study could have been due to a placebo effect (possibly inducing a better "coping strategy") (33, 34). However, recent literature shows that there is little evidence suggesting that a 'fighting spirit' or psychological coping could play an important part in survival (34, 35). First, a review of observational studies showed no relationship between coping style and survival for the large majority of studies (34, 35). Second, a recent meta-analysis (36) of RCTs studying the effect of psychological therapy on survival in women with metastatic breast cancer showed negative results in 4 out of 5 RCTs. Furthermore, as published in this journal (37), cognitive-existential group therapy had no effect on survival even in early-stage breast cancer patients.

In contrast, the present RCT only encompassed a physical intervention (i.e. a series of 8 weekly ATP infusion) which was, indeed, potentially stressful for patients. Moreover, the degree of personal interaction with the research team, including the standard dietary advice provided to all study participants, was identical in the ATP and control group. Also the notion that, in the present study, the favorable effect of ATP on survival was more pronounced in weight-stable patients and in patients with lung cancer, argues against such a placebo effect. Altogether, it would appear highly unlikely that the observed survival benefit of ATP was caused by a placebo effect, especially at 6 months follow up, when any placebo effect would long have faded out.

In conclusion, we found a significant survival benefit of weekly 8-10 hour ATP infusions over a period of 8 weeks, at a maximum dose of 50 µg/kg·min, in pre-terminal cancer patients. In combination with the earlier report by Agteresch et al. (18), who observed a favorable effect of ATP infusions on survival in stage IIIB NSCLC patients, our data corroborate the notion that ATP may increase survival in specific subgroups of cancer patients, especially patients with less advanced disease. Studies are urgently needed to further define the effect of ATP on tumor growth and survival in specific types of cancer, including combination of ATP with curative treatment.

### References

1. Strasser F, Bruera ED: Update on anorexia and cachexia. Hematol Oncol Clin North Am 16:589-617, 2002
2. Walsh D, Donnelly S, Rybicki L: The symptoms of advanced cancer: relationship to age, gender, and performance status in 1,000 patients. Support Care Cancer 8:175-179,2000
3. Dewys WD, Begg C, Lavin PT, et al: Prognostic effect of weight loss prior to chemotherapy in cancer patients. Eastern Cooperative Oncology Group. Am J Med 69:491-497, 1980
4. Bosaeus I, Daneryd P, Lundholm K: Dietary intake, resting energy expenditure, weight loss and survival in cancer patients. The Journal of nutrition 132:3465S-3466S, 2002 (suppl)
5. Vigano A, Bruera E, Jhangri GS, et al: Clinical survival predictors in patients with advanced cancer. Arch Intern Med 160:861-868, 2000
6. Stone PC, Lund S: Predicting prognosis in patients with advanced cancer. Ann Oncol 18:971-976, 2007
7. Maltoni M, Caraceni A, Brunelli C, et al: Prognostic factors in advanced cancer patients: evidence-based clinical recommendations-a study by the Steering Committee of the European Association for Palliative Care. J Clin Oncol 23:6240-6248,2005
8. Warren S: The immediate cause of death. Am J Med Sci 184:610-615, 1932
9. Mantovani G, Maccio A, Massa E, et al: Managing cancer-related anorexia/cachexia. Drugs 61:499-514, 2001
10. Tisdale MJ: Biology of cachexia. J Natl Cancer Inst 89:1763-1773, 1997
11. Argiles JM, Busquets S, Moore-Carrasco R, et al: Targets in clinical oncology: the metabolic environment of the patient. Front Biosci 12:3024-3051, 2007
12. Baracos VE: Cancer-associated cachexia and underlying biological mechanisms. Annu Rev Nutr 26:435-461, 2006
13. Tisdale MJ: Cancer cachexia: metabolic alterations and clinical manifestations. Nutrition 13:1-7, 1997
14. Tisdale MJ: Clinical anticachexia treatments. Nutr Clin Pract 21:168-174, 2006
15. Yavuzsen T, Davis MP, Walsh D, et al: Systematic review of the treatment of cancer-associated anorexia and weight loss. J Clin Oncol 23:8500-8511, 2005
16. Agteresch HJ, Dagnelie PC, van Der Gaast A, et al: Randomized clinical trial of adenosine 5'-triphosphate in patients with advanced non-small-cell lung cancer. J Natl Cancer Inst 92:321-328, 2000
17. Agteresch HJ, Rietveld T, Kerkhofs LG, et al: Beneficial effects of adenosine triphosphate on nutritional status in advanced lung cancer patients: a randomized clinical trial. J Clin Oncol 20:371-378, 2002
18. Agteresch HJ, Burgers SA, van der Gaast A, et al: Randomized clinical trial of adenosine 5'-triphosphate on tumor growth and survival in advanced lung cancer patients. Anticancer Drugs 14:639-644, 2003
19. Swennen EL, Bast A, Dagnelie PC: Immunoregulatory effects of adenosine 5'-triphosphate on cytokine release from stimulated whole blood. Eur J Immunol 35:852-858, 2005
20. Swennen EL, Bast A, Dagnelie PC: Purinergic receptors involved In the immunomodulatory effects of ATP in human blood. Biochem Biophys Res Commun 348:1194-1199, 2006
21. Beijer S, Gielisse EA, Hupperets PS, et al: Intravenous ATP infusions can be safely administered In the home setting: a study in pre-terminal cancer patients. Invest New Drugs 25:571-579, 2007
22. Stratton R, Green CJ, Elia M: Disease-related malnutrition: An evidence-based approach to treatment. Wallingford, CT, Oxford University Press, 2003
23. Ravasco P, Monteiro-Grillo I, Vidal PM, et al: Nutritional deterioration in cancer: the role of disease and diet. Clin Oncol (R Coll Radiol) 15:443-450, 2003
24. Fordy C, Glover C, Henderson DC, et al: Contribution of diet, tumour volume and patient-related factors to weight loss in patients with colorectal liver metastases. Br J Surg 86:639-644, 1999
25. Palomares MR, Sayre JW, Shekar KC, et al: Gender influence on weight-loss pattern and survival of nonsmall cell lung carcinoma patients. Cancer 78:2119-2126, 1996
26. Ravasco P, Monteiro-Grillo I, Camilo M: How relevant are cytokines in colorectal cancer wasting? Cancer J 13:392-398, 2007
27. Correale P, Tagliaferri P, Guarrasi R, et al: Extracellular adenosine 5' triphosphate involvement in the death of LAK-engaged human tumor cells via P2X-receptor activation. Immunol Lett 55:69-78, 1997
28. Rapaport E, Fishman RF, Gercel C: Growth inhibition of human tumor cells in soft-agar cultures by treatment with low levels of adenosine 5'-triphosphate. Cancer Res 43:4402-4406, 1983
29. Schafer R, Hartig R, Sedehizade F, et al: Adenine nucleotides inhibit proliferation of the human lung adenocarcinoma cell line LXF-289 by activation of nuclear factor kappaB1 and mitogen-activated protein kinase pathways. Febs J 273:3756-3767,2006
30. Conigrave AD, van der Weyden L, Holt L, et al: Extracellular ATP-dependent suppression of proliferation and induction of differentiation of human HL-60 leukemia cells by distinct mechanisms. Biochemical pharmacology 60:1585-1591, 2000
31. Agteresch HJ, van Rooijen MHC, van den Berg JWO, et al: Growth inhibition of lung cancer cells by adenosine-5'-triphosphate. Drug Dev Res 60:196-203, 2003
32. Mendoza E, Fosella F, Haskell C, et al: Adenosine triphosphate (ATP) for advanced non-small cell lung cancer (NSCLC): A Phase II multicenter study. Proc Annu Meet Am Soc Clin Oncol 15:A1 238, 1996 (abstr)
33. Grulke N, Bailer H: Fighting spirit-a key to survival in cancer patients?. MMW Fortschr Med 149:35-36, 2007
34. Petticrew M, Bell R, Hunter D: Influence of psychological coping on survival and recurrence in people with cancer: systematic review. Bmj 325:1066-1075, 2002
35. Stephen JE, Rahn M, Verhoef M, et al: What is the state of the evidence on the mind-cancer survival question, and where do we go from here? A point of view. Support Care Cancer 15:923-930, 2007
36. Edwards AG, Hailey S, Maxwell M: Psychological interventions for women with metastatic breast cancer. Cochrane Database Syst Rev. 2:CD004253, 2004
37. Kissane DW, Love A, Hatton A, et al: Effect of cognitive-existential group therapy on survival in early-stage breast cancer. J Clin Oncol 22:4255-4260, 2004.

## Claims

1. Use of ATP for the preparation of a medicament for treating, alleviating and/or enhancing survival of pre-terminal cancer patients.

2. Use according to claim 1 wherein the medicament is in the form of an intravenous infusion.
